# EUROPEAN PATENT APPLICATION

(11) **EP 4 342 967 A1**
(43) Date of publication of application: **27.03.2024**
(21) Application number: 22794367.7
(22) Date of filing: 14.03.2022
(51) Int. Cl.: C11B 1/04, C11B 3/06, C11B 3/10

(54) **SUBSTANCE AHVING HIGH ACTIVITY AND HIGH SAFETY, PREPARATION METHOD THEREFOR, AND APPLICATION THEREOF IN DRUG**

(30) Priority: 30.04.2021 CN 202110479720
(71) Applicant: Suzhou Haiyi Biomedical Technology Co., Ltd, Suzhou, Jiangsu 215421 (CN)
(72) Inventor: ZHU, Yixin, Kunming, Yunnan 650231 (CN); XU, Shuang, Kunming, Yunnan 650231 (CN)
(74) Representative: Winter, Brandl - Partnerschaft mbB
(86) International application number: PCT/CN2022/080584
(87) International publication number: WO 2022/227889

(57) **Abstract**

This invention discloses a highly active and highly safe substance, its preparation method, and its use. This substance is prepared from rubber seed crude oil, through alkali neutralization, centrifugal separation, adsorption filtration, and deodorization. This substance is not only highly active in preventing and treating atherosclerosis than that of rubber seed crude oil; but also highly safe which can meet requirements in the national standard of food safety. The substance can be used in pharmaceuticals to prevent and treat atherosclerosis in patients with cardiovascular and cerebrovascular diseases.

## Description

### Technical Field

The present invention belongs to the field of bio-pharmaceuticals.

### Technical Background

Drugs can have certain side effects, but it is desirable for the side effects to be as minimal as possible, especially when treating chronic diseases. Because patients with chronic diseases need to take drugs for a long time, accumulation of minor side effects over time may also cause significant problems. Atherosclerosis, which leads to cardiovascular and cerebrovascular diseases, is a typical chronic disease with a long course, and patients need to take medicine for life. If a drug with high medicinal activity on atherosclerosis and together with high safety can be developed, it will be a good thing for patients suffering from cardiovascular and cerebrovascular diseases.

Cardiovascular and cerebrovascular diseases caused by atherosclerosis are the first cause of death in China, with great harm, but there is currently no particularly effective drug to prevent and treat atherosclerosis. At present, the drugs for treating atherosclerosis are divided into six categories: cholesterol-lowering drugs, antiplatelet drugs, anticoagulant and thrombolytic drugs, antioxidant drugs, and anti-inflammatory drugs. However, these drugs can only play the role of slowing down the formation of atherosclerosis and stabilizing the atherosclerotic plaque, but cannot completely reverse and ablate the atherosclerotic plaque, so the therapeutic effects are limited.

Medical research (Document 1. Liu Chaoran, Chen Guozhen, Li Yunshan, et al. Research on the regression of atherosclerosis in monkeys [J]. Science China (Part B), 1987 (02): 66-74; Document 2. Liu Chaoran, Yang Liang, Chen Guozhen, et al. Study on the effect of rubber seed oil on atherosclerosis -- I. Effect on the formation and regression of experimental aortic atherosclerosis in rabbits [J] Journal of Kunming Medical College, 1980 (3); Document 3. Liu Chaoran, Li Yunshan, Chen Guozhen, et al Effect of rubber seed oil on changes of blood lipids and heart function during atherosclerosis modeling in monkeys [J] Journal of Tropical Crops, 1985 (2): 1; Document 4. Liu Chaoran, Chen Guozhen, Li Yunshan, Chen Longshun, Tang Chaocai, Zhou Shuyun, Zhang Zhixiong. Research on the preventive effect of rubber seed oil on atherosclerosis in monkeys [J]. Journal of Kunming Medical College, 1985 (02): 8-22. Document 5; Liu Hanjun, Zhang Hongfei, Liu Aiwu, etc Ultrastructural observation of rubber seed oil in preventing experimental atherosclerosis in monkeys [J] Journal of Kunming Medical University, 1986 (2)) shows that rubber seed oil has obvious effects on preventing and treating atherosclerosis, and has the potential to be developed into drugs.

Document 1-5 speculates that rubber seed oil is rich in unsaturated fatty acids and has the effect of reducing blood lipids, and speculates that the effect of reducing blood lipids leads to its reversal and regression effect on atherosclerosis. But it is only speculation after all, which can neither confirm nor deny the relationship between the unsaturated fatty acids contained in rubber seed oil and the effect of reversing atherosclerosis. Up to now, no research has revealed the relationship between the specific components contained in rubber seed oil and its medicinal activity (reversing and ablating the atherosclerotic).

Documents 1-5, as research in the medical field, do not mention the preparation method of rubber seed oil. According to the publication date of the document and the actual situation in China at that time, as well as our survey and investigation, the rubber seed oil in these documents should be the crude oil of rubber seeds in food science, that is, the crude extract of lipid-soluble components of rubber seeds in Plant chemistry.

Although rubber seed crude oil has significant medicinal activity of reversing and ablating atherosclerosis confirmed by animal experiments, it contains many harmful, even toxic ingredients. Both Document 6 (Document 6: Luo Xiaolan, Zhu Wenxin, He Jian, et al. Research and Practice of Rubber Seed Oil Refining [J]. Grain and Oil Processing, 2008, 000(011): 46-49.) and Document 12 (Document 12: Li Linkai, Li Chen, Tao Yin. Research on Short Range/Molecular Distillation Process of Rubber Seed Oil [J]. Grain and Oil Processing (Electronic Version), 2015 (09): 26-28.) indicate that rubber seed crude oil has a high acid value and is rich in impurities. In addition to impurities commonly found in crude vegetable oil such as mechanical impurities, phospholipids, mucus, and glycolipids, it also contains some special impurities such as rubber, resin, lipoprotein, cyanide glycosides, etc., and even produces metal ion chelates due to its strong corrosiveness.

In food science, the process of removing harmful components from vegetable oils is called oil refining. Some papers in the field of food science have studied various refining methods for rubber seed crude oil. Here are these research papers:
Document 6: Luo Xiaolan, Zhu Wenxin, He Jian, et al Research and Practice on Refining Rubber Seed Oil [J] Grain and Oil Processing, 2008 000(011): 46-49
Document 7: Shen Shandeng, Experimental Study on Physical Refining of Rubber Seed Oil [J] Chinese Oil and Fat, 1992 (06): 18-20
Document 8: Hu Xiaohong, Liu Dachuan, Zhang Xincai, Research on the preparation and refining process of rubber seed oil [J] Chinese Oil and Fat, 2005 (11): 66-68
Document 9: Wu Weizhong, The industrial use of rubber seed oil [J] Chinese Oil and Fat, 1988 (05): 62-63
Document 10: Jia Wei, Rubber seed oil production process and practice [J] Chinese Oil and Fat, 2006 (02): 12-14
Document 11: Zu Tingyue, Study on aqueous enzymatic extraction of rubber seed oil and preparation of its micro lotion [D] Jiangnan University, 2013.
Document 12: Li Linkai, Li Chen, Tao Yin, Research on Short Range/Molecular Distillation Process of Rubber Seed Oil [J] Grain and Oil Processing (Electronic Version), 2015 (09): 26-28
Document 13: Guo Xiong, Study on the Preparation of Rubber Seed Oil and Protein [D] Wuhan Light Industry University, 2018
Document 14: Lan Qinmu, Storage Test Report on Refined Rubber Seed Oil [J] Oil Technology, 1982 (05): 12-17
Document 15: Tian Hua, Huang Tao, Su Minghua, Research on the process of rubber seed oil degumming and decolorization [J] Journal of Wuhan Light Industry University, 2007, 26 (002): 9-11
Document 16: Wang Xiaoli, Zhan Lin, Research on the Refining of Rubber Seed Oil [J] Chinese Oil and Fat, 2000, 25 (004): 10-11

These refining methods are different, and the refined oil obtained can be good or bad from food science perspective. The judgment of refining methods in food science often considers the following aspects:
1. Food safety. Can food safety meet the requirements in GB 2716-2018 National Food Safety Standard (Vegetable Oil) of edible oil;
2. Yield. The yield of finished products is the final percentage of weight that can be obtained based on the weight of raw materials. Of course, the higher the yield, the better;
3. Cost. The production cost mainly includes the complexity of the process, the level of equipment requirements, the length of the production cycle, the amount of energy and auxiliary material consumption, and so on. Of course, the more economical, the better.

Although these refining methods have many differences in food science, they have one thing in common. These methods have not considered the medicinal activity of the products obtained. We do not know whether the refined rubber seed oils prepared by these different refining methods still have the effects of reversing and ablating atherosclerosis as described in documents 1-5.

In order to investigate the effect of these refining methods on the biological activity of rubber seed oil (reversal and ablation of atherosclerosis), the present invention refined the rubber seed crude oil according to the method described in documents 6-15 and obtained the corresponding refined oil. It was found that they had almost no pharmacological activity, as detailed in Examples 4-9, no matter whether the safety reaches the standard, the yield is high, and the production cost is low.

Rubber seed crude oil has the medicinal activity of reversing and ablating atherosclerosis, but it also contains a lot of harmful and toxic components; After removing these harmful and toxic components through oil refining, the refined oil also loses its medicinal activity, which is a major problem restricting the development and utilization of rubber seed oil.

### Brief Summary of the Invention

In order to solve the problem of developing and utilizing rubber seed oil, this invention focuses on the medicinal activity of rubber seed oil, while also taking into account the safety requirements in food science.

One purposes of this invention is to provide a completely new refined rubber seed oil that, while meeting the safety requirements of edible oil, has biological activity that can reach and even exceed crude oil.

The second purpose of this invention is to provide a preparation method for this highly active and safety substance.

The third purpose of this invention is to provide use of this highly active and safety substance in pharmaceuticals. This substance can be used in pharmaceuticals to prevent and treat atherosclerotic cardio and cerebrovascular disease.

In order to achieve the above-mentioned objectives, this invention provides the following technical solutions:
A highly active and safety substance is prepared from rubber seed crude oil through alkali neutralization, centrifugal separation, adsorption filtration, and deodorization.

This invention also provides a preparation method for highly active and safety substance, the method comprises: alkali neutralization, centrifugal separation, adsorption filtration, and deodorization, wherein rubber seed crude oil is used as raw material.

The alkali is an edible grade alkali aqueous solution, the number of moles of alkali is equal to the number of moles of fatty acids contained in the rubber seed crude oil.

Centrifugal separation is carried out above 70 °C and centrifugal force is greater than or equal to 5000g.

In order to achieve the conditions for centrifugal separation in large-scale industrial production, this invention also provides a detailed method: the centrifugal separation is carried out in two stages, the first stage is carried out in a tubular centrifuge, and the second stage is carried out in a disc centrifuge or a tubular centrifuge. The first stage of centrifugal separation in a tube centrifuge is carried out under the following conditions: the temperature of centrifugal separation is 70-95 °C, centrifugal force is 5000-30000g, preferably 6000-18000g, then discarding fatty acid salts, and separating the oil phase; The second stage of centrifugal separation using a disc centrifuge or a tube centrifuge is carried out under the following conditions: the temperature of the oil phase substance obtained in the first step is maintained above 70 °C, the centrifugal force is 5000-30000g, preferably 6000-18000g, the fatty acid salt is discarded, and the oil phase is further separated.

The oil phase substance is adsorbed and filtered, deodorized, and finally retained as the finished product.

This invention also provides a pharmaceutical composition, the composition comprises a therapeutically amount of the highly active and safety refined rubber seed oil and a pharmaceutically acceptable carrier.

The invention also provides the use of the highly active and highly safe refined rubber seed oil for the pharmaceutical preparations for the preventing and treating of atherosclerotic cardiovascular and cerebrovascular diseases,wherein the highly active and highly safe substance acts as sole active ingredient in the medicaments.

As compared with prior arts, the rubber seed oil obtained by using the method of the present invention possesses fundamental difference in activity, and also possesses fundamental difference in preparation method.

Based on the experimental data, the present invention has the following advantages:
The present invention provides a new substance whose safety is up to the requirements in the standard( GB 2716-2018 national food safety standard (vegetable oil)) (Example 1-2), and its activity for preventing and curing atherosclerosis can be up to or even surpass that of rubber seed crude oil (Example 10-11).

This invention also provides a method for industrialized and large-scale production of this highly active and safety substance, rather than being limited to laboratory preparation (Examples 1-2).

Substantial contents of the present invention are further described below through the accompanying drawings and examples of the present invention, but the present invention is not limited thereto.

### Descriptions of the Drawings

Figure 1 is the histogram of atherosclerotic lesion grade of each experimental group provided in Example 9.
Figure 2 is the drawing of distribution of atherosclerotic plaques in rabbit aorta provided in Example 10, taken from the normal diet group, the high fat diet group, the rubber seed crude oil + high fat diet group, the present invention + high fat diet group, and the simvastatin + high fat diet group, respectively, from left to right.
Figure 3 is the percentage chart of atherosclerotic plaque in aortic area of each experimental group provided in Example 10.
Figure 4 is the P-value chart of differences between different groups provided in Example 10, P less than 0.05 is a significant difference, P less than 0.01 is an extremely significant difference.
Figure 5 is the histogram of percentage of atherosclerotic plaque in aortic area of each experimental group provided in Example 10.
Figure 6 is the typical drawing of distribution of atherosclerotic plaques in rabbit aorta provided in Example 11, taken from the normal diet group, the atherosclerotic model, the model + normal feeding group, the model + crude oil treatment group, the model + the present invention treatment group, and the model + simvastatin treatment group in sequence from left to right.
Figure 7 is the percentage chart of atherosclerotic plaque in aortic area of each experimental group provided in Example 11.
Figure 8 is the P-value chart of differences between different groups provided in Example 11, P less than 0.05 is a significant difference, P less than 0.01 is an extremely significant difference.
Figure 9 is the histogram of percentage of atherosclerotic plaque in aortic area of each experimental group provided in Example 11.

### Examples

### Example 1. Prepare highly active and highly safe rubber seed oil according to the method of this invention.

Using 1000kg rubber seed crude oil as raw material, the measured acid value is 30mg KOH/g, and based on this, 21.4kg of solid NaOH is needed. Dissolve 21.4kg of solid alkali in 78.6kg of water and prepare 100kg of alkali solution with a weight percentage concentration of 21.4%. Mix the crude oil and alkaline solution in a ratio of 10:1, heat to 80 °C, and then inject them into a tube centrifuge for continuous separation at a centrifugal force of 13000g. Adjust the flow rate to ensure that the mixture stays in the centrifuge for 1.5 minutes. After being buffered, the separated oil phase is heated to 90 °C and injected into a disc centrifuge for further centrifugation. The centrifugal force is 8000g, and the residence time in the centrifuge is 1 minute. After centrifugal separation, the oil phase is subjected to vacuum heating and dehydration, with a vacuum pressure of 10kPa and a heating temperature of 100 °C for 60 minutes. Then, an adsorbent (activated clay) with an oil weight of 1.2% is added. Stir for 10 minutes, and then filter to obtain the clear oil. Heat the clear oil to 150 °C, under a vacuum environment of 1 kPa, introduce water vapor, deodorize for 30 minutes, then stop, cool down, and release pressure to obtain a finished product of 660kg, with a yield of 66%. The product has a light-yellow color and is a clear and transparent oily liquid without foreign objects. It has the inherent odor and taste of rubber seed oil and has no odor.

The finished products obtained have been tested in accordance with safety requirements in the standard(GB 2716-2018 food safety national standard (vegetable oil)) for edible oil and are all qualified. The following are some of the test results in table 1.

**Table 1 Test results of the finished product**

| Project | Requirement | Detection result | Determine |
|---|---|---|---|
| Color and luster | Has the color that the product should have | Light yellow, the color that rubber seed oil should have | qualified |
| Taste and odor | Has the taste and odor that the product should have, without burnt odor, rancidity, and other odors | Has the inherent odor and taste of rubber seed oil, without any other odors | qualified |
| State | The product should be in its proper state, and there should be no foreign objects visible to normal vision | Clear and transparent oily liquid without foreign objects | qualified |
| Acid value KOH/(mg/g) | ≤ 3 | 0.3 | qualified |
| Peroxide value g/100g | ≤ 0.25 | 0.01 | qualified |
| Solvent residue mg/kg | ≤ 20 | not detected | qualified |
| Total arsenic in As mg/kg | ≤ 0.1 | not detected | qualified |
| Lead in Pb mg/kg | ≤ 0.1 | not detected | qualified |
| Aflatoxin B1 ug/kg | ≤ 10 | not detected | qualified |
| Benzo (a) pyrene ug/kg | ≤ 10 | not detected | qualified |
| 467 pesticides residues | GB 2763-2019 | not detected | qualified |

This indicates that the safety of the refined rubber seed oil of this invention has met the safety requirements in the standard (GB 2716-2018 food safety national standard (vegetable oil)) for edible oil.

### Example 2. Prepare highly active and highly safe rubber seed oil according to the method of this invention.

Using 1000 kg rubber crude seed oil as raw material, the measured acid value is 30mg KOH/g, and based on this, 21.4 kg of solid NaOH is needed. Dissolve 21.4 kg of solid alkali in 78.6kg of water and prepare 100kg of alkali solution with a weight percentage concentration of 21.4%. Mix crude oil and alkaline solution in a ratio of 10:1, heat to 95 °C, and then inject them into a tube centrifuge for continuous separation at a centrifugal force of 18000g. Adjust the flow rate so that the mixture stays in the centrifuge for 1.5 minutes. After buffering the separated oil phase, adjust the temperature to 70 °C, and inject it into the tubular centrifuge to continue centrifugal separation. The centrifugal force is 6000g, and the centrifugal residence time is 1.5 minutes. After centrifugal separation, the oil phase is subjected to vacuum heating and dehydration, with a vacuum pressure of 10kPa and a heating temperature of 100 °C for 60 minutes. Then, an adsorbent (a mixture of activated clay and diatomaceous earth) with an oil weight of 1.5% is added. The mixture is stirred for 10 minutes, and then filtered to obtain the clear oil. Heat the clear oil to 160 °C, under a vacuum environment of 0.5kPa, introduce water vapor, deodorize for 30 minutes, then stop, cool down, and release pressure to obtain a finished product of 650kg, with a yield of 65%. The product has a light-yellow color and is a clear and transparent oily liquid without foreign objects. It has the inherent odor and taste of rubber seed oil and has no odor.

The finished product obtained has been tested in accordance with the safety requirements in the standard (GB 2716-2018 food safety national standard (vegetable oil)) for edible oil and are all qualified. The following in table 2 are some of the test results.

**Table 2 Test results of the finished product**

| Project | Requirement | Detection result | Determine |
|---|---|---|---|
| Colour and lustre | Has the color that the product should have | Light yellow, the color that rubber seed oil should have | qualified |
| Taste and odor | Has the taste and odor that the product should have, without burnt odor, rancidity, and other odors | Has the inherent taste and odor of rubber seed oil, without any other odors | qualified |
| State | The product should be in its proper state, and there should be no foreign objects visible to normal vision | Clear and transparent oily liquid without foreign objects | qualified |
| Acid value KOH/(mg/g) | ≤ 3 | 0.6 | qualified |
| Peroxide value g/100g | ≤ 0.25 | 0.02 | qualified |
| Solvent residue mg/kg | ≤ 20 | not detected | qualified |
| Total arsenic in As mg/kg | ≤ 0.1 | not detected | qualified |
| Lead in Pb mg/kg | ≤ 0.1 | not detected | qualified |
| Aflatoxin B1 ug/kg | ≤ 10 | not detected | qualified |
| Benzo (a) pyrene ug/kg | ≤ 10 | not detected | qualified |
| 467 pesticides residues | GB 2763-2019 | not detected | qualified |

This indicates that the safety of the refined rubber seed oil of this invention has met the safety requirements in the standard (GB 2716-2018 food safety national standard (vegetable oil)) for edible oil.

### Example 3. Explore the conditions and methods for achieving centrifugal separation i n large-scale industrial production.

3.1. Centrifuge directly with a disc centrifuge. Using 1000kg rubber seed crude oil as raw material, the measured acid value is 30mg KOH/g, and based on this, 21.4kg of solid NaOH is needed. Dissolve 21.4kg of solid alkali in 78.6kg of water and prepare 100kg of alkali solution with a weight percentage concentration of 21.4%. The crude oil and alkaline solution are mixed in a ratio of 10:1, heated to 80 °C, and then injected into a disc centrifuge for centrifugation, but the separation failed. No matter how to adjust the flow rate, change the ratio of crude oil to alkaline solution, adjust centrifugal force, adjust temperature and other technical parameters, all have failed.

3.2 Centrifuge directly with a tube centrifuge. Using 1000kg rubber seed crude oil as raw material, the measured acid value is 30mg KOH/g, and based on this, 21.4kg of solid NaOH is needed. Dissolve 21.4kg of solid alkali in 78.6kg of water and prepare 100kg of alkali solution with a weight percentage concentration of 21.4%. Mix the crude oil and alkaline solution in a ratio of 10:1, heat to 80 °C, and then inject them into a tube centrifuge for continuous separation at a centrifugal force of 10000g. Adjust the flow rate to ensure that the mixture stays in the centrifuge for 1.5 minutes. The separated oil phase was subjected to vacuum heating and dehydration, followed by adsorption filtration, but the filtration failed. No matter how the adsorbent composition, adsorbent dosage, temperature, pressure and other conditions of the filtration are changed, all filtering attempts have failed.

3.3. Optimize the centrifugal force of the first step of centrifugation. Using 1000kg rubber seed crude oil as raw material, the measured acid value is 30mg KOH/g, and based on this, 21.4kg of solid NaOH is needed. Dissolve 21.4kg of solid alkali in 78.6kg of water and prepare 100kg of alkali solution with a weight percentage concentration of 21.4%. Mix crude oil and alkaline solution in a ratio of 10:1, heat to 80 °C, and then inject them into a tube centrifuge. From 1000g to 40000g, gradually increase the centrifugal force at intervals of 1000g, and it is found that effective separation cannot be achieved when the centrifugal force is below 5000g. When the centrifugal force is above 5000g (including), effective separation can be achieved,with an increase in centrifugal force and an increase in equipment processing efficiency. When the centrifugal force exceeds 30000g, there is a significant increase in equipment material requirements, equipment accuracy requirements, equipment wear and tear, and equipment operational difficulties, but the benefits obtained are limited. The comprehensive performance is optimal when the centrifugal force is between 6000-18000g.

3.4. Optimize the centrifugal force of the second step of centrifugation. Using 1000kg rubber seed crude oil as raw material, the measured acid value is 30mg KOH/g, and based on this, 21.4kg of solid NaOH is needed. Dissolve 21.4kg of solid alkali in 78.6kg of water and prepare 100kg of alkali solution with a weight percentage concentration of 21.4%. Mix crude oil and alkaline solution in a ratio of 10:1, heat to 80 °C, and then inject them into a tube centrifuge for continuous separation at a centrifugal force of 15000g. After being buffered, the separated oil phase is heated to 95 °C and injected into a tube or disc centrifuge for further centrifugation. From 1000g to 40000g, gradually increase the centrifugal force at intervals of 1000g. It is found that effective separation cannot be achieved when the centrifugal force is below 5000g, but can be achieved when the centrifugal force is above 5000g (including); The increase in centrifugal force increases the processing efficiency of the equipment. When the centrifugal force exceeds 30000g, there is a significant increase in equipment material requirements, equipment accuracy requirements, equipment wear and tear, and equipment operational difficulties, but the benefits obtained are limited. The comprehensive performance is optimal when the centrifugal force is between 6000-18000g.

### Example 4. Prepare refined rubber seed oil according to the method in document 6.

Using 1000kg rubber seed crude oil as raw material, the measured acid value is 30 mg KOH/g. According to the optimization method in Document 6, adjust the oil temperature to 40°C, add 3kg of 85% phosphoric acid, mix thoroughly, and then raise the temperature to 80 °C. Quickly stir, add 80kg softened water (water from which calcium and magnesium ions are removed) at 80°C, and change to slow stirri ng, raise the temperature to 85 °C, then continue stirring for 30 minutes, stop stirri ng, let it settle for more than 6 hours, release the precipitated gum, sewage, and t hen perform vacuum dehydration. The vacuum dehydration pressure is 0.005MPa, an d the temperature is 90 °C. After dehydration, the oil temperature is cooled to bel ow 40 °C under vacuum. Heat the pre-decolorized oil to 110 °C, and under a vacuu m pressure of 0.005MPa, add 25kg of activated clay, while adding 1.25kg of activat ed carbon to the clay. When the re-decolorization filtration is halfway through, switc h to pre-decolorization of the oil. When the pressure of the blade filter reaches the upper limit of the equipment, stop filtering and blow out the shaking cake, a filter ing cycle is completed. The entire filtering process is very difficult and the filtering speed is very slow. The decolorized oil enters the deacidification tower with a press ure of less than or equal to 100Pa. The temperature of the oil entering the tower is heated to 240-250 °C, and the outlet temperature is controlled at ≥ 230 °C. The acid value of the oil at the outlet is controlled at ≤ 1mgKOH/g. After cooling the oi I to room temperature, 810kg of refined oil product was obtained using the method described in document 6, with a yield of 81%. The refined oil appears red, clear, t ransparent, and odorless.

The refined oil is tested according to the safety requirements in the standard (GB 2716-2018 food safety national standard (vegetable oil)) for edible oil, and is determined to be a qualified product.

### Example 5. Refined rubber seed oil is prepared according to the method in docume nt 10.

Using 1000kg rubber seed crude oil as raw material, the measured acid value is 30mg KOH/g. According to the method in document 10, heat the oil temperature to 60 °C, add 2kg of 85% phosphoric acid, react for 18 minutes, let it settle, release the lower liquid, wash with water once, and obvious emulsification occurs. Heat the oil to 65 °C, add 1kg formic acid, react for 20-25 minutes, let it settle, and wash with water 3 times. Each time, obvious emulsification occurs, and the oil turns milky white. Under vacuum conditions with a pressure of 0.6 kPa, heat the oil to 112 °C for dehydration. After dehydration, add 40kg of activated clay and 10 kg of activated carbon, and stir at a temperature of 100 °C for 30 minutes. Then filter while it is hot to remove the adsorbent. Filtering is quite difficult and the filtering speed is very slow. The filtered clear oil is extracted in a ratio of oil: alcohol=1:1.5, and 75% ethanol is added for the first round. It is mixed and stirred at a temperature of 58 °C. Let the mixture stand for layering, and then separate the lower oil phase. Then proceed with the second round of extraction according to the conditions of the first round; Repeat this process for 15 rounds of extraction, with an acid value below 3, and then end the extraction. The oil phase is heated under negative pressure and ethanol is evaporated to obtain 690kg of refined oil product prepared by the method described in document 10, with a yield of 69%. The refined oil appears red, clear and transparent, and can smell the odor of ethanol after being stored in a container for a long time.

The refined oil is tested in accordance with the safety requirements in the standard (GB 2716-2018 food safety national standard (vegetable oil)) for edible oil, and two indicators are found to be unqualified, while the other indicators are found to be qualified. The unqualified indicators are: taste and odor, solvent residue.

### Example 6. Refined rubber seed oil is prepared according to the method in docume nt 12.

Using 1000g of rubber seed crude oil as raw material, the measured acid value is 30mg KOH/g. According to the optimization method in Document 12, adjust the oil temperature to 40 °C, add 3g of 85% phosphoric acid, mix thoroughly, and then raise the temperature to 80 °C. Quickly stir, add 80g softened water (water from which calcium and magnesium ions are removed) at 80°C, and change to slow stirring, raise the temperature to 85 °C, then continue stirring for 30 minutes, stop stirring, let it settle for more than 6 hours to layer, separate the upper oil layer from the lower precipitated gum and sewage, and then perform dehydration. Under normal pressure, heat the oil to 110 °C until no bubbles emerge. After dehydration, cool the oil to below 40 °C. Heat the oil to 110 °C, add 50g of activated clay, and add 2.5g of activated carbon to the clay. Stir for 30 minutes, and then filter with 3 layers of filter paper. In order to accelerate the filtration speed, the Buchner funnel suction filtration method is used. Nevertheless, the filtering speed is still slow. The filtered oil enters the molecular distillation device for molecular distillation. The conditions for molecular distillation are: the rotation frequency of the feed pump is 20Hz, the speed of the scraping rotor is 300rpm, the coolant temperature is 25 °C, the temperature of the heat transfer oil is 200 °C, and the pressure of the vacuum pump is 1.33Pa.

Directly following the method in document 12 for one round of distillation, the acid value is still as high as 12mgKOH/g, which is higher than the standard of 3mgKOH/g in the safety requirements in the standard(GB 2716-2018 food safety national standard (vegetable oil)) for edible oil. After repeating three rounds of molecular distillation, the acid value decreased to below 3mgKOH/g, resulting in a final product of 830g prepared by the method described in document 12, with a yield of 83%.

The refined oil is tested according to the safety requirements in the standard (GB 2716-2018 food safety national standard (vegetable oil)) for edible oil, and is determined to be a qualified product.

### Example 7. Refined rubber seed oil is prepared according to the method in docume nt 14.

Using 1000kg rubber seed crude oil as raw material, the measured acid value is 30mg KOH/g. Heat the crude oil to 70 °C, add 3kg of 85% phosphoric acid, add 20kg of saturated NaCl aqueous solution, let it stand, separate the lower layer of hydrates, and retain the upper layer of oil phase. Add 21.4kg of solid NaOH to 78.6kg of saturated NaCl solution, and prepare 100kg of NaOH saturated NaCl solution (hereinafter referred to as alkali sodium chloride solution) with a weight percentage concentration of 21.4%. Add 100kg of alkali sodium chloride solution to the oil phase and heat the whole reaction system to 70 °C. At this point, the oil phase and alkali sodium chloride solution are divided into two layers without any acid-base neutralization reaction. In order to allow the oil phase to contact and react with the alkali solution, a slow stirring method is used. The alkali sodium chloride solution gradually contacts with the oil phase and undergoes an acid-base neutralization reaction, producing a large amount of fatty acid sodium. Then, emulsification occurs. Emulsifies the oil phase / sodium fatty acid / alkali sodium chloride solution together, forming a brown, viscous, and uniform emulsification system. Keep temperature and stand for 24 hours, the upper layer of emulsion is taken and washed with saturated saline water at 100 °C, which still shows severe emulsification. There is still no obvious layering after 24h of heat insulting and standing. Take the upper emulsion and heat it to 120 °C for dehydration. Dehydration is extremely difficult, with a large amount of foam overflowing. After dehydration, 120kg of refined oil is obtained using the method described in document 14, with a yield of 12%. The refined oil presents a viscous jelly like state, reddish black, cloudy, and has a significant odor.

The refined oil is tested according to the standard (GB 2716-2018 food safety national standard (vegetable oil)) for edible oil, and is determined as unqualified product.

### Example 8. Refined rubber seed oil is prepared according to the method in docume nt 13.

Using 1000g of rubber seed crude oil as raw material, the measured acid value is 30mg KOH/g. According to the optimization method in document 13, dissolve 1000g of rubber seed crude oil into 1703g of n-hexane, and prepare a 37% mass fraction of rubber seed crude oil n-hexane solution. Select a filter membrane with a pore size of 10kDa and allow the crude oil n-hexane solution to pass through the membrane under a positive pressure of 0.23MPa. By heating the filtered crude oil n-hexane solution and removing the n-hexane solvent under negative pressure, 950 grams of the product prepared by the method described in document 13 is obtained, with a yield of 95%. This product is a dark red, opaque oily liquid that is not significantly different from the raw material of crude oil.

This product is tested according to the standard (GB 2716-2018 food safety national standard (vegetable oil)) for edible oil, and many indicators did not meet the requirements, especially the acid value is up to27mgKOH/g. No matter how many times it is filtered through the membrane, the acid value is still above 25mgKOH/g, and there is no possibility of reducing it to below 3mgKOH/g. The physical and chemical indicators of the substance obtained by this method are not significantly different from that of crude oil.

### Example 9. Test medicinal activities of 6 samples (reversing and ablating atheroscler osis) through animal experiments.

These 6 samples are: rubber seed crude oil, refined rubber seed oil of this invention, and the rubber seed oils prepared in Example 4 (Document 6), Example 5 (Document 10), Example 6 (Document 12), and Example 7 (Document 14). Considering that the physical and chemical indicators of the rubber seed oil prepared in Example 8 (Document 13) are not significantly different from those of the rubber seed crude oil, it is not selected for animal experiments.

Select 12 8-week-old male wild-type C57BL/6J mice; Select 84 8-week-old male APOE -/- mice, weighing 24 ± 2g. APOE -/- mice are randomly divided into 7 groups, with 12 in each group. Add 12 wild-type C57BL/6J mice as an additional group, resulting in a total of 8 groups of mice. The experimental treatment plan for each group of mice is as follows:
(1) Normal diet group (Negative control group, **NC**): 12 8-week-old male wild-type C57BL/6J mice, fed with basic feed and gavage with physiological saline;
(2) High fat diet group (Positive control group, **PC**): 12 APOE -/- mice, fed on a high-fat diet and gavage with peanut oil, with a gavage dose of 6.2g/kg/d;
(3) rubber seed crude oil + high-fat diet group **(CRSO):** 12 APOE -/- mice were fed on a high-fat diet and gavage with rubber seed crude oil at a dose of 6.2g/kg/d;
(4) Present invention + high-fat diet group **(INV):** 12 APOE -/- mice were fed on a high-fat diet and gavage with the preparation in Example 1-2, with a gavage dose of 6.2g/kg/d;
(5) Example 4 + high-fat diet group **(EX4):** 12 APOE -/- mice were fed on a high-fat diet and gavage with the preparation of Example 4 (Document 6), with a gavage dose of 6.2g/kg/d;
(6) Example 5 + high-fat diet group **(EX5):** 12 APOE -/- mice, fed on a high-fat diet and gavage with the preparation of Example 5 (Document 10), with a gavage dose of 6.2g/kg/d;
(7) Example 6 + high-fat diet group **(EX6):** 12 APOE -/- mice, fed on a high-fat diet and gavage with the preparation of Example 6 (Document 12), with a gavage dose of 6.2g/kg/d;
(8) Example 7 + high-fat diet group **(EX7):** 12 APOE -/- mice were fed on a high-fat diet and gavage with the preparation of Example 7 (Document 14), with a gavage dose of 6.2g/kg/d.

Above groups of mice are continuously gavaged for 8 weeks. High fat feed formula: basic feed: 70%, lard: 20%, sucrose: 5%, milk powder: 4%, bile salt: 1%, cholesterol: 0.15%.

The mice are killed, the thorax of the mice is cut, the blood vessels of the mice are perfused with pre-cooled normal saline, the aorta is stripped, cut completely, washed with PBS, fixed in 10% formaldehyde solution, and the atherosclerotic lesion grade is detected by grading method:
Level 0: The surface of the aortic intima is smooth, without changes in cream color, i.e., no plaques;
Level 0.5: There are extensive creamy or milky white changes in the aortic intima, but no patches protruding from the surface;
Level 1: There are obvious cream-colored raised plaques on the aortic intima, with a plaque area of less than 3mm²;
Level 2: There are obvious cream-colored raised plaques on the aortic intima, but there is no fusion into patches. The largest plaque has an area greater than 3mm²;
Level 3: There are many patches of varying sizes, some fused into patches, and the area of large patches exceeds 3mm²;
Level 4: The surface of the arterial intima is almost entirely covered by fused plaques.

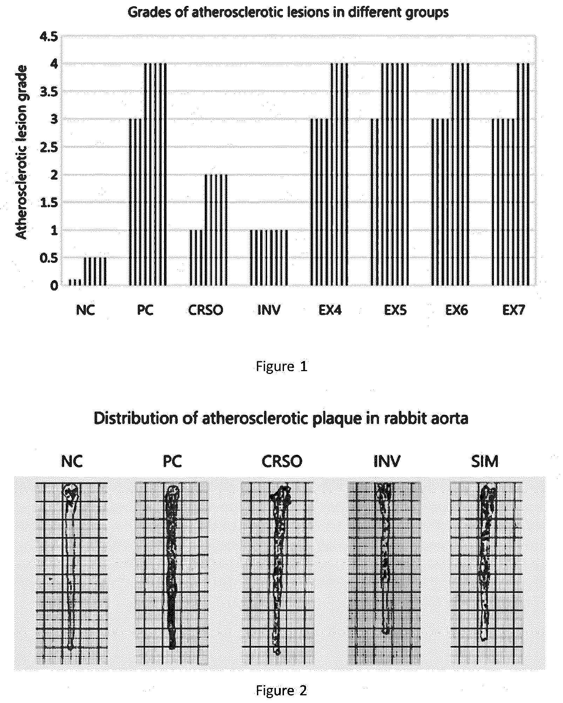

The results of animal experiments are shown in the following table 3 and Figure 1.

**Table 3 Atherosclerotic lesion grade of each experimental group**

| Serial number | Experimental group | Atherosclerotic lesion grade |
|---|---|---|
| 1 | NC (Negative control, Normal diet group) | Level 0-0.5 |
| 2 | PC (Positive control, high-fat diet group) | Level 3-4 |
| 3 | CRSO (rubber seed crude oil + high-fat diet group) | Level 1-2 |
| 4 | INV (Present invention + high-fat diet group) | Level 1 |
| 5 | EX4 (Example 4 + high-fat diet group) | Level 3-4 |
| 6 | EX5 (Example 5 + high-fat diet group) | Level 3-4 |
| 7 | EX6 (Example 6 + high-fat diet group) | Level 3-4 |
| 8 | EX7 (Example 7 + high-fat diet group) | Level 3-4 |

This experiment indicates that:
(1) The refined rubber seed oil of this invention has the medicinal activity of preventing and inhibiting atherosclerotic plaque. After adding the refined rubber seed oil of this invention, the development degree of atherosclerosis in APOE -/- mice fed with high-fat diet is grade 1; The degree of atherosclerosis in positive control group,APOE -/- mice fed with high-fat diet is grade 3-4; After the refined rubber seed oil of this invention is added, the degree of atherosclerosis development in APOE -/- mice is significantly slowed down, which indicates that the rubber seed oil of this invention has the medicinal activity of preventing and inhibiting arteriosclerosis.
(2) The refined rubber seed oils prepared in Examples 4, 5, 6 and 7 have no obvious medicinal activity on preventing and inhibiting atherosclerotic plaque. After adding the rubber seed oils prepared in Example 4 (Document 6), Example 5 (Document 10), Example 6 (Document 12) and Example 7 (Document 14), the development degree of atherosclerosis in APOE -/- mice fed with high-fat diet are also 3-4 levels as those of positive group, indicating that the refined rubber seed oils prepared in Example 4 (Document 6), Example 5 (Document 10), Example 6 (Document 12) and Example 7 (Document 14), have no medicinal activity to prevent and inhibit atherosclerosis.

### Example 10

This animal experiment studies the medicinal activity of three samples to inhibit the formation of atherosclerosis: rubber seed crude oil, refined rubber seed oil of this invention and simvastatin.

Rabbits are ideal animal models for diet induced atherosclerosis research, and their pathogenesis is closer to that of human body as compared with gene deficient mice and rats. Select 40 4-month-old male Japanese white rabbits weighing 2.0 ± 0.2kg and randomly divide them into 5 groups as follows:
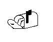 Normal diet group (Negative control group, **NC**): 8 male Japanese white rabbits, fed with basic feed;
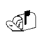 High fat diet group (Positive control group, **PC**): 8 male Japanese white rabbits, fed with peanut oil + high fat fortified basic feed, with a peanut oil feeding dose of 1.5g/kg/d (converted based on adult and animal body weight, body surface area and Km factor);
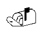 rubber seed crude oil + high-fat diet group **(CRSO):** 8 male Japanese white rabbits, fed with rubber seed crude oil + high fat fortified basic feed, and the feeding dose of rubber seed crude oil is 1.5g/kg/d (converted based on adult and animal body weight, body surface area and Km factor);
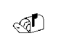 Present invention + high-fat diet group **(INV):** 8 male Japanese white rabbits, fed with the preparation in Example 1-2 + high-fat fortified basic feed, and the feeding dose is 1.5g/kg/d (converted based on adult and animal body weight, body surface area and Km factor);
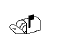 Simvastatin + high-fat diet group **(SIM):** 8 male Japanese white rabbits, fed with simvastatin + high-fat fortified basic feed, with a dosage of 2.0mg/kg/d (converted based on adult and animal body weight, body surface area and Km factor).

High fat fortified feed: Each rabbit is fed with 0.5 grams of cholesterol, 2.0 grams of lard, and 20 grams of egg yolk daily.

Feed for 6 weeks according to the above grouping and feeding conditions, and then execute the animals. After the animals are killed, the aorta is removed from the aortic valve orifice to the beginning of the common iliac artery, the aortic wall is cut longitudinally along the midline of the abdomen, fixed with 10% formaldehyde solution, and the distribution map of aortic lesions is drawn according to the aorta. The area of atherosclerotic plaque and the total area of aortic expansion are measured, and the percentage of plaque area is calculated.

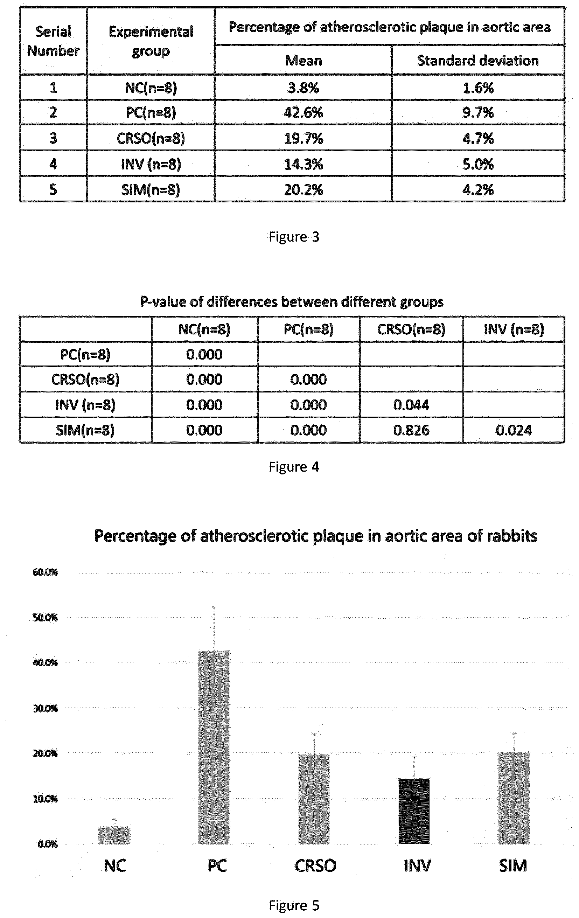

The experimental results are shown in Figure 2, Figure 3, Figure 4, and Figure 5.

The experimental results indicate that:
(1) The substance of this invention has obvious medicinal activity of preventing and inhibiting atherosclerotic plaque (the plaque area of the **INV** group is 14.3 ± 5.0%, which is less than 42.6 ± 9.7% of the **PC** group, P<0.01, the difference is extremely significant);
(2) The medicinal activity of the substance of this invention on preventing and inhibiting atherosclerotic plaque is stronger than that of the rubber seed crude oil (the plaque area of the **INV** group is 14.3 ± 5.0%, which is smaller than that of the **CRSO** group 19.7 ± 4.7%, P<0.05, with significant difference);
(3) The medicinal activity on preventing and inhibiting atherosclerotic plaque of the substance of this invention, at a dose of 1.5g/kg/d, is stronger than that of simvastatin at a dose of 2.0mg/kg/d (the plaque area of the **INV** group is 14.3 ± 5.0%, which is smaller than that of the **SIM** group, 20.2 ± 4.2%, P<0.05, with significant difference).

### Example 11

This animal experiment studies the medicinal activity of three samples for reversing and ablating atherosclerosis: rubber seed crude oil,substance of this invention and simvastatin.

Rabbits are an ideal animal model for diet-induced study of atherosclerosis. Compared with gene-deficient mice and rats, their pathogenesis is closer to humans. Forty-eight 4-month-old male Japanese white rabbits weighing 2.0 ± 0.2kg were selected and randomly divided into 2 groups, as follows:
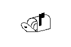 Normal diet group (Negative control group, **NC**): 8 male Japanese white rabbits, fed with basic feed;
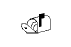 Atherosclerosis modeling group **(MOD):** 40 male Japanese white rabbits, fed with high fat fortified basic diet, and the dose of peanut oil was 1.5g/kg/d (converted based on adult and animal weight, body surface area and Km factor);

High fat fortified feed: Each rabbit is fed with 0.5 grams of cholesterol, 4.0 grams of lard, and 20 grams of egg yolk daily.

According to the above grouping and feeding conditions, one rabbit died in the normal diet group during the 6-week feeding period.

6 weeks later, 8 rabbits in the atherosclerosis modeling group are randomly selected and killed to test whether the atherosclerosis model is established successfully. The test method is as follows: after the animals are killed, the aorta is removed from the aortic valve orifice to the beginning of the common iliac artery, the aortic wall is cut longitudinally along the midline of the abdomen, fixed with 10% formaldehyde solution, and the distribution map of aortic lesions is drawn according to the aorta. The area of atherosclerotic plaque and the total area of aortic expansion is measured, and the percentage of plaque area is calculated. For comparison, 8 rabbits in the normal diet group are also killed to detect the area percentage of aortic atherosclerotic plaque.

After confirming that the rabbits in the atherosclerosis modeling group are successfully modeled, the remaining 32 rabbits in the model group are randomly divided into 4 groups, 8 in each group, as follows:
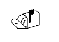 Modeling + basic feeding group **(MBF):** 8 male Japanese white rabbits successfully modeling atherosclerosis are fed with peanut oil and basic diet, and the feeding dose of peanut oil is 1.5g/kg/d (converted according to adult and animal weight, body surface area and Km factor conversion);
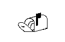 Modeling + rubber seed crude oil treatment group **(CRSO):** 8 male Japanese white rabbits with successful atherosclerosis modeling are fed with rubber seed crude oil and basic feed, and the rubber seed crude oil is fed at a dose of 1.5g/kg/d (converted according to adult and animal weight, body surface area and Km factor conversion);
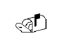 Modeling + present invention treatment group **(INV):** 8 male Japanese white rabbits with successful atherosclerosis modeling are fed with the preparation of Example 1-2 and basic feed, the feeding dose of the preparation of Example 1-2 is 1.5g/kg/d (converted according to adult and animal weight, body surface area and Km factor conversion);
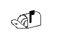 Modeling + simvastatin treatment group **(SIM):** 8 male Japanese white rabbits with successful atherosclerosis modeling are fed with simvastatin and basic diet, and the simvastatin feeding dose is 2.0mg/kg/d.

According to the above grouping and feeding conditions, the feeding is continued for 3 months, during which one rabbit died in **MBF** group and one in **SIM** group. Three months later, all animals are euthanized.

After the animals are killed, the aorta is removed from the aortic valve orifice to the beginning of the common iliac artery, the aortic wall is cut longitudinally along the midline of the abdomen, fixed with 10% formaldehyde solution, and the distribution map of aortic lesions is drawn according to the aorta. The area of atherosclerotic plaque and the total area of aortic expansion is measured, and the percentage of plaque area is calculated.

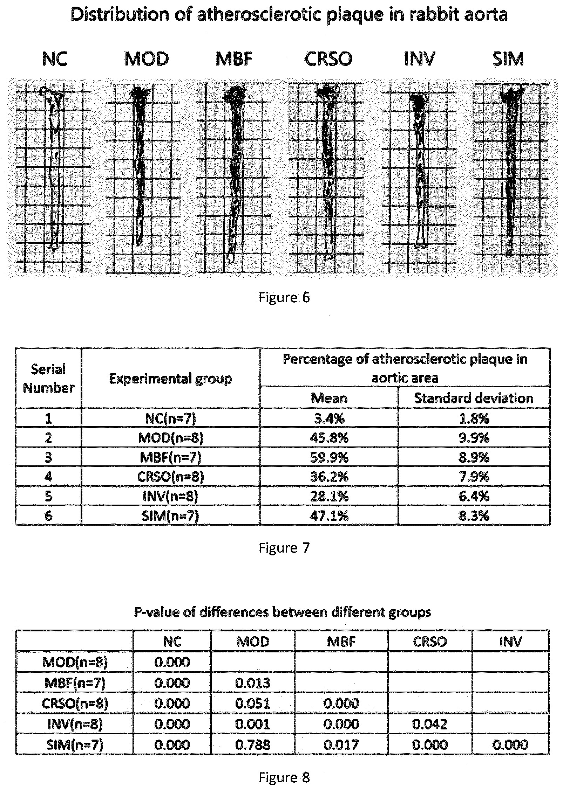

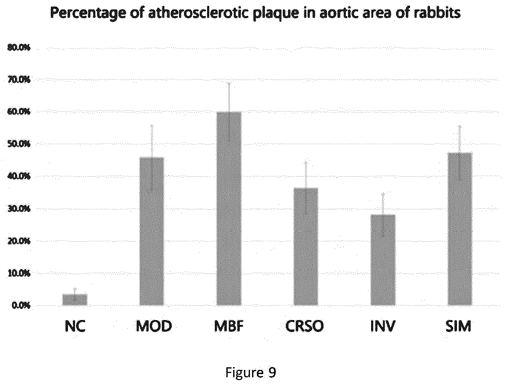

The experimental results are shown in Figure 6, Figure 7, Figure 8, and Figure 9.

The experimental results indicate that:
(1) The substance of this invention can not only inhibit the increasing trend of atherosclerosis (the plaque area of the **INV** group is 28.1 ± 6.4%, which is smaller than 59.9 ± 8.9% of the **MBF** group, P<0.01, the difference is extremely significant), but also can ablate the formed atherosclerosis plaque (the plaque area of the INV group is 28.1 ± 6.4%, which is smaller than 45.8 ± 9.9% of the MOD group, P<0.01, the difference is extremely significant);
(2) The medicinal activity of the substance of this invention in ablating atherosclerotic plaque is better than that of the rubber seed crude oil (the plaque area of the **INV** group is 28.1 ± 6.4%, which is less than 36.2 ± 7.9% of the **CRSO** group, P<0.05, with significant difference);
(3) The substance of this invention at a dose of 1.5g/kg/d has a better medicinal activity on inhibiting atherosclerotic plaque than simvastatin at a dose of 2.0mg/kg/d (the plaque area of the INV group is 28.1 ± 6.4%, less than 47.1 ± 8.3% of the SIM group, P<0.01, with extremely significant difference).

### Example 12

The highly active and safety substance of this invention prepared in Examples 1-2, is added with the excipient in a ratio of 1:1 or 1:2 by weight to produce tablets. Tablets can be used as medicines.

### Example 13

The highly active and safety substance of this invention prepared in Examples 1-2, is wrapped in a soft capsule material and made into a soft capsule according to the conventional capsule preparation method. Soft capsules can be used as medicines.

### Example 14

The highly active and safety substance of this invention prepared in Examples 1-2, is added with an emulsifier, osmotic pressure regulator, and water, and subjected to high-pressure homogenization emulsification to obtain an injection emulsion.

### Example 15

The highly active and safety refined rubber seed oil of this invention prepared in Examples 1-2, is tableted according to the following method:
Tablets:
100mg of refined rubber seed oil of this invention;
Appropriate amount of starch;
Appropriate amount of corn syrup;
Magnesium stearate in moderation.
Preparation method comprises the following steps:
   Mix the refined rubber seed oil of this invention with an auxiliary agent, sieve it, and evenly mix it in a suitable container, and then prepare the resulting mixture into tablets.

### Example 16

Prepare the highly active and safety refined rubber seed oil of this invention prepared in Examples 1-2, into soft capsules according to the following method:
Soft capsule:
1000mg of refined rubber seed oil of this invention;
Appropriate amount of gelatin;
Appropriate amount of glycerol;
Moderate amount of water;
Preparation method comprises the following steps:
   Soak gelatin and glycerol in distilled water to expand and soften the gelatin, then stir and mix evenly to obtain the capsule material glue solution. Take out the prepared capsule material adhesive solution, apply it on a flat surface of the board to ensure uniform thickness, and then heat it at a temperature of about 90 °C to evaporate surface moisture to obtain a soft film with a certain toughness and elasticity. Finally, the refined rubber seed oil (1000mg) of this invention is loaded into the soft capsule skin using a shot pressing mold or an automatic rotating capsule rolling machine to obtain the soft capsule.

### Example 17

Prepare the highly active and safety refined rubber seed oil of this invention prepared in Examples 1-2, into an injection emulsion according to the following method:
Injection emulsion:
200g of refined rubber seed oil of this invention
Moderate amount of lecithin
Moderate amount of glycerol
Moderate amount of sodium chloride
Moderate amount of water
Preparation method:
   According to the formula, refined rubber seed oil of this invention, lecithin, and glycerol, are mixed, water is added to 1000mL, an appropriate amount of sodium chloride is added to adjust the osmotic pressure, high-pressure homogenization emulsification is carried out, sterilization is carried out at 121 °C, and the injection emulsion is obtained.

### Example 18

Tablets: 10mg of refined rubber seed oil of this invention prepared according to the method of Examples 1-2, 180mg of lactose, 55mg of starch, and 5mg of magnesium stearate;
Preparation method: Mix the refined rubber seed oil of this invention, lactose, and starch, evenly moisten with water, sieve and dry the moistened mixture, then sieve, add magnesium stearate, and then press the mixture into tablets. Each tablet weighs 250mg, and the content of the refined rubber seed oil of this invention is 10mg.

### Example 19

Capsules: 2000g of refined rubber seed oil of this invention prepared according to the method of Examples 1-2, 1000g of gelatin, 500g of glycerol, and 1000g of water;
Preparation method: Soak 1000g of gelatin and 500g of glycerol in 1000g of water to expand and soften the gelatin, then stir and mix evenly to obtain the capsule material adhesive solution. Take out the prepared capsule material adhesive solution, apply it on a flat surface of the board to ensure uniform thickness, and then heat it at a temperature of about 90 °C to evaporate surface moisture, becoming a soft film with a certain toughness and elasticity. Finally, 1000mg of refined rubber seed oil of this invention is loaded into the soft capsule skin using a shot pressing mold or an automatic rotating capsule rolling machine to prepare the soft capsule. Each soft capsule weighs 1.4g, and the content of the refined rubber seed oil of this invention is 1000mg.

### Example 20

Injection emulsion: 200g of refined rubber seed oil of this invention prepared according to the method of Examples 1-2, 25g of glycerol, 12g of lecithin, 1000g of water, and 1g of sodium chloride;
Preparation method: Accurately weigh 200g of refined rubber seed oil of this invention, 25g of glycerol, and 12g of lecithin, mix, add water to 1000mL, add sodium chloride, and adjust the osmotic pressure to 350mOsm/kg·H₂O. By high-pressure homogenization and emulsification, the emulsion is obtained, sterilized at 121 °C, cool to room temperature, and filled into a 250mL sterile glass bottle under sterile conditions for encapsulation. The volume of each injection emulsion is 250mL, and the content of the refined rubber seed oil of this invention is 50g.

## Claims

1. A highly active and highly safe substance **characterized in that** the substance is prepared from rubber seed crude oil through alkali neutralization, centrifugal separation, adsorption filtration, and deodorization.

2. A preparation method for a highly active and highly safe substance as claimed in claim 1, **characterized in that** the method comprises: alkali neutralization, centrifugal separation, adsorption filtration, and deodorization, wherein rubber seed crude oil is used as raw material.

3. The preparation method as claimed in claim 2, wherein the alkali is an edible grade alkali aqueous solution, the number of moles of alkali is equal to the number of moles of fatty acids contained in the rubber seed crude oil.

4. The preparation method as claimed in claim 2, wherein the centrifugal separation is carried out above 70 °C and centrifugal force is greater than or equal to 5000g.

5. The preparation method as claimed in claim 4, wherein the centrifugal separation is carried out in two stages: the first stage is carried out in a tubular centrifuge, and the second stage is carried out in a disc centrifuge or a tubular centrifuge.

6. The preparation method as claimed in claim 5, wherein the first stage is carried out in a tubular centrifuge, followed by discarding fatty acid salts, and separating oil phase, temperature of the centrifugal separation is 70-95 °C, centrifugal force is 5000-30000g, preferably 6000-18000g.

7. The preparation method as claimed in claim 5, wherein the second stage of centrifugal separation is carried out in a disc centrifuge or a tube centrifuge, followed by discarding fatty acid salts, and separating oil phase, temperature of the centrifugal separation is above 70 °C, centrifugal force is 5000-30000g, preferably 6000-18000g.

8. A pharmaceutical composition comprises a therapeutically effective amount of a highly active and highly safe substance as claimed in claim 1 and a pharmaceutically acceptable carrier.

9. Use of highly active and highly safe claimed in claim 1 for preparation of medicaments for preventing and treating of atherosclerotic cardiovascular and cerebrovascular diseases.

10. The use according to claim 10, wherein the highly active and highly safe substance acts as sole active ingredient in the medicaments.
